# EUROPEAN PATENT SPECIFICATION

(11) **EP 0 442 851 B1**
(45) Date of publication and mention of the grant of the patent: **14.12.1994**
(21) Application number: 91830040.1
(22) Date of filing: 11.02.1991
(51) Int. Cl.: A61B 17/32, A61F 9/00, A61B 17/22

(54) **A surgical instrument having interchangeable cutting blades**
Chirurgisches Instrument mit auswechselbaren Schneidklingen
Instrument chirurgical avec des lames échangeables

(30) Priority: 14.02.1990 IT 4762990
(43) Date of publication of application: 21.08.1991
(73) Proprietor: Caponi, Mauro, I-00148 Roma (IT)
(72) Inventor: Caponi, Mauro, I-00148 Roma (IT)
(74) Representative: Iannone, Carlo Luigi

(56) References cited:
- EP-A- 0 174 084
- EP-A- 0 217 368
- US-A- 3 902 498
- US-A- 4 577 629

## Description

This invention relates to a surgical instrument having interchangeable cutting blades.

More particularly, this invention relates to a surgical instrument of thealternate, pneumatically driven type, which is of such structure as to allow the substitution of the cutting tools to be performed, so that the instrument can be employed again a number of times, and can be sterilized in an autoclave.

Instruments of the kind dealt with in this invention have been already known for many years.

More particularly, the Italian Patent No. 1,101,754 describes an alternate linear stroke instrument for surgical use, and in particular for ocular surgery, in which instrument a pneumatic power is employed which causes the cutting tool or the cutting blade to shift, and an elastic member for causing said cutting blade to go back or return.

A similar solution has been suggested by the European Patent Application No. 0174084, in which a surgical instrument is disclosed, in particular for percutaneous disectomy, having functional and structural characteristics substantially equal to those of the Italian Patent mentioned above.

This last mentioned kind of instrument, in its configuration available on the market, does not allow either the front small tube or cannula of the instrument or the cutting blade of the sane to be substituted.

Moreover, such instruments, and in particular that of the EP-A-0,174,084, are realized according to the most widespread philosophies of the "single use", so that they can be employed just for a staple surgical operation.

This type of features limits the adaptability of the instrument in employing the same, as it is not possible to adapt such instrument to different surgical requirements due to the impossibility of substituting the cutting tools, and further the fact that the instrument can be used just once increases the costs of the surgical operation very much, as the unit cost of such surgical instruments is very high.

In the light of what has been mentioned above, it is quite evident that it would be advantageous to have at disposal a surgical instrument of said kind, said instrument being of the alternate, pneumatically driven type, and allowing both the front small tube or cannula and the cutting blade to be substituted, which blade can be thus changed according to specific surgical requirements, or for a further operation, said instrument being further so realized as to allow its sterilization to be carried out in an autoclave or by means of any other procedure, and to be employable again.

These and other results are obtained according to the present invention through the suggestion of a surgical instrument of the kind mentioned above in which a system is provided on its front portion for realizing the coupling of the outer cannula or small tube; and in which the possibility is supplied at the back portion of inserting the cutting blade and of coupling the same in a fixed way to the member that is shifted alternately by a pneumatic driving action. The features of the preamble portion of claim 1 are known from US-A-4,577,629.

Accordingly, it is a specific object of the present invention a surgical instrument having a body provided with an inlet opening for a driving gas, a small hollow tube or cannula, which is provided in its front portion with an opening and is arranged in front of said body; a movable member which is positioned inside the body of the instrument and which can shift alternately when it is driven by said driving gas and is counteracted in its motion by counteracting means, and a cutting blade which is hollow inside and is movable, its motion being integral with the motion of said movable member and whose front end is positioned inside said outer small tube or cannula and said blade is provided with a front opening and is connected at its back portion to a suction system, and said outer small tube or cannula is coupled at the front portion of said body through a removable first coupling means, and said cutting blade is adapted to be inserted into said body at the back portion, and being characterized in that a guide is integrally coupled with said movable member, said cutting blade being also provided at the back portion of said body with a second removable coupling means, which couples with corresponding coupling means, which are borne by said cutting blade so as to allow the motion of the blade itself to occur integrally with said movable member, and that said body is further provided with an inlet opening for introducing a physiological solution.

The whole surgical instrument according to this invention is realized with a sterilizable material. More particularly, the movable member or barrel is made up of titanium.

The coupling means between the outer cannula and the body, and between said guide and the cutting blade, will preferably be made up of a threaded tap-and female coupling, or of a fixed joint or of a bayonet joint.

A spacing member can be provided between the two coupling members between said guide and said cutting blade.

A groove can be provided in said movable member, said groove becoming coupled with a reference dowel arranged inside the body, in order to prevent the movable member itself from rotating.

Again according to the present invention, the body can be realized as formed by two disassemblable parts so that the maintenance of the same can be performed.

Means can be provided on the back part of the body itself for adjusting the position of said guide and hence of said cutting blade.

The driving gas will preferably be supplied at an adjustable pulsating frequency.

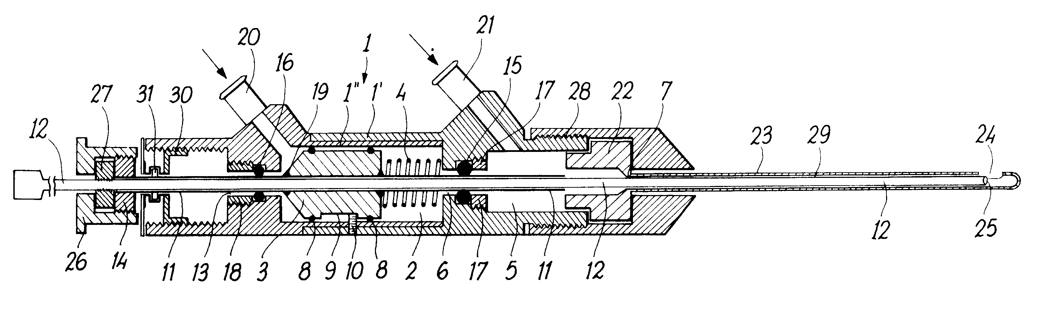

This invention will be disclosed in the following with particular reference to a preferred embodiment of the same which is shown in the enclosed drawing, wherein a longitudinal cross-sectional view is shown of the surgical instrument in question.

The instrument according to the present invention, which can be employed as already mentioned above for different times of surgical operations, is made up of a body 1, which comprises two parts 1′ and 1˝ that can be disassembled for their maintenance, inside which body a first chamber 2 is realized which houses a barrel 3 as well as a counteracting spring 4 arranged in front of said barrel 3.

In said body 1, a second chamber 5 is realized in front of said first chamber 2, said second chamber being connected to the first one by means of an opening 6 and being provided in the front portion with a hole 7.

Sealing gaskets 8 are provided on said barrel 3, a groove 9 being also provided which prevents the barrel from rotating by coupling with a reference dowel 10.

A channel or guide 11 for the passage of the cutting blade 12 is provided through the barrel 3, said spring 4, said opening 6 and said second chamber 5, said channel going, at the back part of the barrel 3 and through a hole 13, towards the outer portion of the body and ending in a threading 14.

Sealing means 15 and 16 are provided in the coupling between the opening 6 and the guide 11, as well as between the hole 13 and the guide 11, said sealing means being kept in position by threaded metal rings 17 and 18 which are coupled with the opening 6 and the hole 13. Moreover, said guide 11 is welded at the point 19 to said barrel 3.

An inlet 20 for the pneumatic action gas is realized in the body 1 at the joint corresponding to said first chamber 2, at the back of the barrel 3, whereas an inlet 21 is provided at the point corresponding to the second chamber 5 for the introduction of the physiological solution.

The member 22 which is so shaped as to strike against the body 1 and which is integral with the cannula 23 and externally coaxial with said blade 12, is arranged in front of said body 1.

Said small tube or cannula 23 is provided in the front position with the opening 24 whereas the blade 12, which is hollow in its central portion, is provided with openings 25.

The blade 12 is inserted into the body 1 of the instrument according to the present invention at the back portion, through said channel or guide 11, the diameter of said blade being such that, once the insertion has been performed, a gap is left between said blade 12 and the cannula 23.

The blade 12 is fastened at the back portion to said channel or guide 11, on the threading 14, by means of the threaded metal ring 26. A spacer 27, which is borne by said blade 12, is arranged between said threaded metal ring 26 and the threading 14.

The blade 12 is connected at the back portion to a suction system (not shown) through which the matter cut by means of the cutting blade is sucked.

The threading 28 which is provided between two portions of the body 1, allows the assembly consisting of the member 22-cannula 23 to be locked in the front position between two said portions.

The thrust of the gas which is introduced at an adjustable pulsating frequency through the inlet 20, pushes in the forward direction the barrel 3 which is counteracted and then returned to the starting position by the spring 4, so that the alternate motion of the cutting blade 12 is obtained, because it is integral with the guide 11, which is in turn welded to the barrel 3.

The physiological solution which is introduced through the inlet 21, is inserted into the gap 29 between the blade 12 and the cannula 23.

A vernier 30 is provided on the inside part of the back end of the body 1, from which end the guide 11 projects, said vernier, by becoming coupled with a relieved part 31 borne by said guide 11, allowing the position of the front end of the blade 12 to be adjusted with respect to the opening 25 of the cannula 23.

The instrument realized according to the present invention gives the possibility of substituting the small tube or cannula 23 and the blade 12 so that the instrument itself can be re-employed and adapted to a number of different requirements regarding surgical operations, by assembling tools of different sizes.

Moreover, by realizing the barrel 3 according to the shape disclosed herein and employing a sterilizable material, and in particular employing titanium, it is possible to proceed to the integral sterilization of the surgical instrument according to the present invention in an autoclave.

This invention has been disclosed just for illustrative and not for limitative purposes, according to some preferred embodiments of the same, but it is to be understood that modifications and/or changes can be introduced within the scope of the appended claims.

## Claims

1. A surgical instrument having a body (1) which is provided with an inlet opening (20) for a driving gas, a hollow small tube or cannula (23) which is provided in its front portion with an opening (24) and is arranged in front of said body; a movable member (3) which is positioned inside the body of the instrument and can shift alternately, when driven by said driving gas, which is counteracted in its motion by counteracting means (4), and a cutting blade (12) which is hollow inside and is movable according to a motion integral with that of said movable member, said cutting blade having its front end positioned inside said outer cannula and said blade is provided with a front opening (25) and is connected at its back portion to a suction system; and said outer cannula (23) is coupled at the front portion of said body through a removable first coupling means, and said cutting blade is adapted to be inserted into said body at the back portion, and being characterized in that a guide (11) is coupled integrally (19) with said movable member, which guide is provided at the back portion of said body with a second removable coupling means, which couples with corresponding coupling means borne by said cutting blade, so as to allow the blade itself to move integrally with the movable member, and that said body is further provided with an inlet opening (21) for the introduction of a physiological solution.

2. A surgical instrument according to claim 1, characterized in that it is made up of a sterilizable material

3. A surgical instrument according to claim 1, characterized in that said movable member is made up of titanium.

4. A surgical instrument according to any one of the preceding claims, characterized in that the coupling means between said cannula and the body are made up of a threaded tap-female coupling, or of a fixed joint or of a bayonet joint.

5. A surgical instrument according to anyone of the preceding claims 1-3, characterized in that the coupling means between the guide and the cutting blade are made up of a threaded tap-female coupling, of a fixed joint or of a bayonet joint.

6. A surgical instrument according to claim 5, wherein a spacing member is arranged between said coupling means provided on the guide as well as on the cutting blade.

7. A surgical instrument according to any one of the preceding claims, characterized in that a groove is provided in said movable member, which groove couples with a reference dowel arranged inside the body, in order to avoid the rotation of the movable member.

8. A surgical instrument according to any one of the preceding claims, characterized in that said body is made up of two parts which can be disassembled.

9. A surgical instrument according to anyone of the preceding claims, characterized in that means are provided on the back part of said body for adjusting the position of the cutting blade with respect to said cannula.

10. A surgical instrument according to anyone of the preceding claims, wherein said driving gas is introduced according to an adjustable pulsating frequency.

## Patentansprüche

1. Chirurgisches Instrument mit auswechselbaren Schneidkligen, welches einen, mit einer Einlassoeffnung (20) Euer ein Treibgas versehenen Koerper (1), ein kleines, an seiner Sitrnseite mit einer Oeffnung (24) versehenes und gegenueber dem vorgenannten Koerper angeodnetes Hohlrohr oder Kanuele (23); einen verstellbaren, innerhalb des Koerpers des Instruments angeordneten Teil (3), der hin- und her- bewegbar und in seiner Bewegung durch ein Widerstandsmittel (4) gehindert ist, und eine hoehle, mit der Bewegung des vorgenannten verstellbaren Teiles integral bewegbare Schneidklinge (12) aufweist, wobei diese Schneidklinge mit einer stirnseitigen Oeffnung (25) versehen und an seiner Rueckseite an eine Sauganordnung verbunden ist und die vorgenannte aeussere Kanuele (23) an die Stirnseite des vorgenannten Koerpers durch ein loesbares erstes Kupplungsmittel angekuppelt ist und wobei die vorgenannte Schneidklinge in den Rueckteil des vorgenannten Korper einsetzbar ist, dadurch gekennzeichnet, dass eine Fuehrung (11) an den vorgenannte verstellbare Teil integral (19) gekuppelt ist und an der Rueckseite des vorgenannten Koerpers mit einem loesbaren, zweiten Kupplungsmittel versehen ist, das an entsprechendes, durch die vorgenannte Schneidklinge getragenes Kupplungsmittel angekuppelt ist, so dass die Klinge selbst sich integral mit dem verstellbaren Teil zu bewegen vermag, und dass der vorgenannte Koerper ausserdem mit einer Einlassoeffnung (21) zur Einfuehrung einer physiologischen Loesung versehen ist.

2. Chirurgisches Instrument nach Anspruch 1, dadurch gekennzeichnet, dass es aus einem Sterilisierbaren Werkstoff hergestellt ist.

3. Chirurgisches Instrument nach Anspruch 1, dadurch gekennzeichnet, dass der vorgenannte verstellbare Teil aus Titan hergestellt ist.

4. Chirurgisches Instrument nach je einem der vorhergehenden Ansprueche, dadurch gekennzeichnet, dass die Kupplungsmittel zwischen der vorgenannte Kanuele und dem vorgenannten Koerper aus einer geschnittenen Nut- und Feder-Kupplung oder einer festen Kupplung oder auch aus einer Bajonett-Kupplung besteht.

5. Chirurgisches Instrument nach je einem der vorhergehenden Ansprueche 1 bis 3, dadurch gekennzeichnet, dass die Kupplungsmittel zwischen der Fuehrung und der Schneidklinge aus einer geschnittenen Nut- und Feder-Kupplung, einer festen Kupplung oder einer Bajonett-Kupplung bestehen.

6. Chirurgisches Instrument nach dem Anspruch 5, worin zwischen den vorgenannten Kupplungsmitteln auf der Fuehrung sowie auf der Schneidklinge ein Abstandshalter angeordnet ist.

7. Chirurgisches Instrument nach je einem der vorhergehenden Ansprueche, dadurch gekennzeichnet, dass im vorgenannten verstellbaren Teil eine Nut vorgesehen ist, in welche ein innerhalb des Koerpers angeordnete Stift eingreift, um die Drehung des verstellbaren Stiftes zu verhindern.

8. Chirurgisches Instrument nach je einem der vorhergehenden Ansprueche, dadurch gekennzeichnet, dass der vorgenannte Koerper aus zwei voneinander entfernbaren Teilen besteht.

9. Chirurgisches Instrument nach je einem der vorhergehenden Ansprueche, dadurch gekennzeichnet, dass auf der Rueckseite des vorgenannten Koerpers Mittel zur Einstellung der Lage der Schneidklinge zur vorgenannten Kanuele vorgesehen sind.

10. Chirurgisches Instrument nach je einem der vorhergehenden Ansprueche, worin das vorgenannte Treibgas entsprechend einer einstellbaren Pulsfrequenz zugefuehrt wird.

## Revendications

1. Instrument de chirurgie ayant un corps (1), qui est muni d'une ouverture d'entré (20) pour un gaz propulsif, un petit tube creux ou canule (23), qui est muni à sa partie frontale d'une ouverture (24) et est placé devant dudit corps; un élément mobile (3), qui est positioné au devant du corps de l'instrument et peut se déplacer alternativement, quand actioné par ledit gaz propulsif, dont mouvement est opposé par de moyens d'opposition (4), et une lame d'incision (12) cave, qui est mobile selon un mouvement intégral de ce dudit élément mobile, ladite lame d'incision ayant son bout frontal placé dans ladite canule et ladite lame étant muni d'une ouverture frontale (25) et étant jointe à sa partie postérieure avec un système d'aspiration et ladite canule extérieure (23) est enclenchée à la dite portion frontale dudit corps par un premier moyen d'accouplement découplable et ladite lame d'incision est apte à être insérée dans ledit corps à la partie postérieure et étant caractérisé en ce que un chemin (11) est accouplé integralement avec ledit élément mobile, ce chemin étant muni à la partie postérieure dudit corps d'un deuxiéme moyen d'accouplement découpable, qui s'accouple avec des moyens d'accouplement correspondants supportés par ladite lame d'incision, de façon à permettre à ladite lame de se déplacer intégrallement avec l'élément mobile et en ce que ledit corps est muni aussi d'une ouverture d'entré (21) pour l'introduction d'une solution physiologique.

2. Instrument de chirurgie selon la revendication 1, caractérisé en ce qu'il est fait d'un matériel stérilisable.

3. Instrument de chirurgie selon la revendicatin 1, caractérisé en ce que ledit élément mobile est fait de titane.

4. Instrument de chirurgie selon une quelconque des revendications précédantes, caractérisé en ce que lesdits moyens d'accouplement entre ladite canule et le corps sont faits d'un joint fileté à tenon et mortaise ou d'un joint fixe ou d'un joint à baïonette.

5. Instrument de chirurgie selon une quelconque des revendications précédantes, caracterisé en ce que lesdits moyens d'accouplement entre le chemin et la lame d'ncision sont faits d'un joint fileté à tenon et mortaise ou d'un joint fixe ou d'un joint à baïonette.

6. Instrument de chirurgie selon la revendication 5, dans lequel un élément-entretoise est placé entre lesdits moyens d'accouplement prévus sur le chemin ainsi que sur la lame d'incision.

7. Instrument de chirurgie selon une quelconque des revendications précédentes, caractérisé en ce que une rainure est prévue dans ledit élément mobile pour s'accoupler avec un goujon de référence placé dans le corps, afin d'empêcher la rotation de l'élément mobile.

8. Instrument de chirurgie selon une quelconque des revendications précédentes, caractérisé en ce que ledit corps est fait en deux parties, qui peuvent être désassamblées.

9. Instrument e chirurgie selon une quelconque des revendications précédentes, caractérisé en ce que des moyens sont prevus à la partie postérieure dudit corps pour ajuster la position de la lame d'incision au regard de ladite canule.

10. Instrument e chirurgie selon une quelconque des revendications précédentes, dans lequel ledit gaz de propulsion est introduit selon une fréquence de pulsation ajustable.
